# EUROPÄISCHE PATENTSCHRIFT

(11) **EP 2 579 085 B1**
(45) Veröffentlichungstag und Bekanntmachung des Hinweises auf die Patenterteilung: **13.02.2019**
(21) Anmeldenummer: 12187618.9
(22) Anmeldetag: 08.10.2012
(51) Int. Cl.: G01J 1/42

(54) **Flexibles nichtlineares Laserscanning Mikroskop zur nicht-invasiven dreidimensionalen Detektion**
Flexible non-linear laser scanning microscope for non-invasive three-dimensional detection
Microscope à balayage laser flexible pour la détection non invasif à trois dimensions

(30) Priorität: 08.10.2011 DE 102011115944
(43) Veröffentlichungstag der Anmeldung: 10.04.2013
(73) Patentinhaber: JenLab GmbH, 07745 Jena (DE)
(72) Erfinder: KÖNIG, Karsten, 66119 Saarbrücken (DE); WEINIGEL, Martin, 07745 Jena (DE)
(74) Vertreter: Freitag, Joachim

(56) Entgegenhaltungen:
- EP-A1- 0 154 866
- DE-A1-102009 029 831
- DE-U1-202010 009 249
- GB-A- 2 068 597
- JP-B2- 2 606 227
- US-A- 4 659 916
- US-A1- 2007 020 785

## Beschreibung

Die Erfindung betrifft ein nichtlineares Laserscanning-Mikroskop zur flexiblen nicht-invasiven dreidimensionalen Detektion, insbesondere in lebender Materie (in vivo), vorzugsweise in der Haut von Tieren, in Pflanzen und Zellkulturen, sowie in nicht-lebender Materie, wie beispielsweise Mineralien, Textilien und Kunstwerken. Unter einem nichtlinearem Laserscanning-Mikroskop bzw. Mikroskop mit Multiphotonen-(MP)-Anregung soll im Folgenden stets ein Mikroskop mit Zwei- oder Mehrphotonen-Anregung verstanden werden.

Es sind Zweiphotonen-Mikroskope und Multiphotonentomographen (MPT) bekannt, bei denen Fluoreszenz- und SHG-Signale (Second Harmonic Generation) in biologischen Molekülen in vivo durch gepulste Laserstrahlung im nahen Infrarotbereich angeregt und durch geeignete hochempfindliche und schnelle Empfänger detektiert werden (z. B. US 5 034 613 A, DE 201 17 294 U1; DE 203 06 122 U1). Zudem können nicht-fluoreszierende und nicht SHG-aktive Komponenten wie Wasser- und Lipide mittels CARS-Mikroskopen und CARS-Tomographen (CARS - Coherent Anti-Stokes Raman Scattering) dargestellt werden (siehe z.B. nicht vorveröffentlichte DE 10 2010 047 578 A1).

Mit diesen nichtlinearen optischen Mikroskopen und Tomographen werden horizontale Auflösungen im Submikrometerbereich und Scanbereiche (Objektbereiche) von einigen hundert Mikrometern in drei Dimensionen erreicht.

Derartige nichtlineare Mikroskope und Tomographen basieren auf der Verwendung von starren Aufbauten und schwingungsgedämpften optischen Tischanordnungen. Sie können entweder als aufrechte oder inverse optische Mikroskopanordnungen betrieben werden. Es sind jedoch keine hochauflösenden bildgebenden Systeme (mit lateraler Auflösung um 1 µm und darunter) für die nichtlineare Mikroskopie oder Tomographie bekannt, die bei der geforderten lateralen und axialen Auflösung im Sub-Mikrometerbereich eine freie Positionierung des Messkopfes gestatten.

Eine dafür erforderliche Strahlübertragung kann durch optische Fasersysteme oder als Freistrahlübertragungssystem realisiert werden. Die Genauigkeit von Freistrahl-Laserübertragungssystemen, wie diese beispielsweise in Form von Spiegelgelenkarmen bekannt sind, wird durch mechanische Abweichungen, mechanische Spannungen und Temperaturdrift innerhalb der mechanischen Käfigstruktur dieser Arme beeinflusst. Der Einfluss auf die mechanische Achse des Spiegel-Gelenkarmes (beispielsweise durch Temperaturdrift) führt bei unterschiedlichen Konstellationen des Gelenkarmes zu einer Abweichung der Position der Laserstrahlung am Ausgang des Gelenkarmes gegenüber der Zielposition bzw. der mechanischen Achse, sodass eine zuverlässige Abtastung eines Messobjekts mittels hochauflösender Fokussieroptik unter unterschiedlichen Messkopfausrichtungen nur eingeschränkt möglich ist. Die Schwankungen der Position der Laserstrahlung am Ausgang des optischen Gelenkarmes, können mit dem Begriff "Genauigkeit des Übertragungssystems" zusammengefasst werden.

Weiterhin wird die Genauigkeit der Laserstrahlübertragung durch die gegebene Länge des optischen Strahlenganges eines Gelenkarmes und die Strahlpositionsstabilität (engl. pointing stability) des Lasers beeinflusst.

Als Pointing stability soll hierbei die Änderung der Richtungsstabilität der Laserstrahlung am direkten Ausgang des Lasers bezeichnet werden, die aus thermisch bedingter Beeinflussung der Resonatorkonfiguration des Lasers resultieren.

Die Einkopplung der Laserstrahlung in einen Spiegel-Gelenkarm ist besonders kritisch. Eine optimale Einkopplung ist gegeben, wenn die optische Achse der Laserstrahlung mit der mechanischen (Eintritts-) Achse des Gelenkarmes übereinstimmt (Kollinearität). Die hierbei auftretenden Abweichungen beeinflussen die Genauigkeit zusätzlich zu den oben benannten mechanisch und thermisch bedingten Abweichungen des Gelenkarmes selbst.

Bei der Verwendung von Faserübertragungsoptiken reduzieren sich die zu kompensierenden Abweichungen in der Strahlführung zwar ausschließlich auf die Strahlein- und -auskopplung, jedoch bleibt das Grundproblem der unerwünschten Laserstrahlabweichung am Zielort dasselbe. Insbesondere besteht auch die Gefahr einer unerwünschten Einkopplung in den Fasermantel ("cladding").

Aus der US 5,463,215 A ist eine Vorrichtung zur Ausrichtung eines Lichtstrahls zum Zwecke seiner Einkopplung in eine optische Faser bekannt, bei der ein Teil des Laserstrahls, der nicht in den Faserdurchmesser eingekoppelt wird, über zwei ringförmige konische Spiegel, die als Retroreflektoren angeordnet sind, sowie einen auf einem Ring unter 45° rotierenden Spiegel auf eine Detektoreinrichtung ausgekoppelt wird. Durch Synchronisation des Detektors mit der Position des umlaufenden Spiegels kann dann eine Abweichung von der Kollinearität von Laser- und Faserachse sowie eine Defokussierung erkannt werden. Außerdem ist eine überdeckende Beleuchtung des Fasereingangs (d.h. Strahldurchmesser größer als Faserdurchmesser) erforderlich und somit ein Intensitätsverlust bereits von Anfang an unvermeidlich.

Aus der DE 20 2010 009 249 U1 ist eine Anordnung zur MPT-Bildgebung bekannt, die über einen flexiblen optischen Gelenkarm eine im Raum freie Positionierung eines Messkopfes gewährleistet, wobei der Messkopf die Optik zur Strahlführung und Photonendetektion enthält. Durch eine aktive Strahljustage mit Online-Kontrolle soll eine optimale Bildqualität in jeder Messkopflage erreichbar sein. Über die Art und Weise der Strahljustage sind jedoch keinerlei Angabe gemacht, sodass ein selbsttätig messendes System zur Strahlstabilisierung nicht zur Verfügung steht.

Aus dem Stand der Technik ergeben sich somit folgende Nachteile:
- für die Umsetzung eines flexiblen Mikroskops ist die Strahlstabilisierung wegen der mechanischen Toleranzen der strahlführende Systeme zu groß, um die ausreichende Konstanz der Ausleuchtung eines Messflecks zur reproduzierbaren Bestrahlung des Scanareals für eine Submikrometer-Auflösung durch visuelle Beobachtung und online-Nachstellung zu gewährleisten, und
- bei der Multiphotonenmikroskopie ist die Beschränkung der Bildgebung auf die Detektion der Autofluoreszenz körpereigener Stoffe und auf nichtlineare Frequenzvervielfachung bestimmter Moleküle nicht ausreichend, wobei die wünschenswerte Kombination mit CARS-Systemen wegen der hohen Kollinearitätsanforderungen bisher nur in starren Systemen realisiert werden konnte.

Der Erfindung liegt deshalb die Aufgabe zugrunde, eine Realisierung für ein mobiles, flexibel einsetzbares nichtlineares Laserscanning-Mikroskop zur nicht-invasiven dreidimensionalen Detektion für multivalente hochauflösende nichtlineare Untersuchungen, insbesondere von Haut, Geweben im Urogenitalbereich, in der Mundhöhle und im Augenbereich des menschlichen Körpers, aber auch an Pflanzen, Tieren, in Zellkulturen und in nicht-lebender Materie, zu finden, die eine automatische reproduzierbare Ausleuchtung der Fokussieroptik bei unbeschränkter Flexibilität des Messkopfes gestattet. Als erweiterte Aufgabenstellung soll eine reproduzierbar übereinstimmende gleichzeitige Applikation von zwei Strahlengängen zum Zwecke der Kombination von unterschiedlichen Messverfahren, vorzugsweise von MP- und CARS-Tomographie, in einem gemeinsamen flexiblen Messkopf realisierbar sein.

Erfindungsgemäß wird die Aufgabe durch ein flexibles, nichtlineares LaserscanningMikroskop gemäß Anspruch 1 gelöst. Vorteilhaft ist der mindestens eine steuerbare Kippspiegel mittels mindestens eines Spiegelhalters auf Basis eines Antriebsprinzips aus der Gruppe von kapazitiven, induktiven oder Piezo-Stellern, Schrittmotor oder Gleichstrommotor angetrieben.

Der mindestens eine steuerbare Kippspiegel kann dabei zweckmäßig mittels eines zweiachsigen oder mittels zweier einachsiger Spiegelhalter realisiert werden.

Als Zielposition für die stabilisierte Ausrichtung des mindestens einen Anregungsstrahls wird vorteilhaft die Eintrittspupille der Fokussieroptik oder eines anderen aperturbegrenzten optischen Elements gewählt.

Es erweist sich als zweckmäßig, dass der Strahlteiler zur Erzeugung des Teststrahls in Abhängigkeit von der Strahlführung des mindestens einen Anregungsstrahls im Messkopf entweder als Reflexions- oder Transmissionsstrahlteiler ausgebildet ist. Der Strahlteiler zur Auskopplung des Teststrahls kann vorteilhaft zugleich als dichroitischer Spiegel zur kollinearen Einkopplung zweier unterschiedlicher Anregungsstrahlen ausgebildet sein.

Die Photodiode zur Detektion des Teststrahls als Äquivalent des Anregungsstrahls in der Zielposition ist vorzugsweise eine kreisrunde Photodiode. Alternativ sind jedoch auch rechteckige oder andere Detektorformen einsetzbar. Vorteilhaft kann mit der Photodiode zusätzlich zur Regelung der Strahlstabilität in der Übertragungsoptik die Pointing-Stabilität des Lasers bestimmt und korrigiert werden. Das Detektorsystem für die aus dem Messobjekt emittierte Sekundärstrahlung ist vorzugsweise mit einem Detektor zur MPT- oder SHG-Bildgebung ausgebildet ist, sodass ein flexibles Diagnosesystem für fluoreszierende Substanzen vorhanden ist, das über einen flexiblen optischen Gelenkarm geringer mechanischer Genauigkeit eine beliebig freie Positionierung des Messkopfes im Raum gestattet und eine reproduzierbare MPT-Bildgebung gewährleistet.

Das Detektorsystem für die Sekundärstrahlung kann besonders vorteilhaft mit mehreren Detektoren zur MPT-, SHG- und zur CARS-Bildgebung ausgebildet sein, sodass ein flexibles Diagnosesystem für fluoreszierende und nichtfluoreszierende Substanzen in lebender Materie vorhanden ist, das über einen gemeinsamen flexiblen optischen Gelenkarm eine beliebig freie Positionierung des gemeinsam genutzten Messkopfes im Raum gestattet und eine gleichzeitige und ortsgleiche Bildgebung aus MPT-, SHG- und CARS-Signalen erzeugt. Dabei kann das so kombinierte Diagnosesystem für fluoreszierende und nichtfluoreszierende Substanzen auch zweckmäßig über einen flexiblen optischen Gelenkarm und eine separate optische Faser die beliebig freie Positionierung des gemeinsam genutzten Messkopfes im Raum gestatten. Des Weiteren ist es möglich, bei dem flexiblen kombinierten Diagnosesystem über zwei separate optische Fasern eine beliebig freie Positionierung des gemeinsamen Messkopfes zur gleichzeitigen und ortsgleichen Bildgebung zu realisieren.

Ferner kann das Detektorsystem für die Sekundärstrahlung in einer kompakten Variante einen gemeinsamen Messdetektor zur MPT-Bildgebung und zur CARS-Bildgebung aufweisen, wobei Mittel zur zeitweisen Unterbrechung der CARS-Anregung vorgesehen sind, die eine Separierung der MPT-Bildgebung gegenüber einer kombinierten Signaldarstellung erlauben.

Vorzugsweise weist das Detektorsystem für eine zeitkorrelierte Einzelphotonenzählung TCSPC-Detektoren zur MPT-Bildgebung auf. Dabei ist das Detektorsystem zweckmäßig mit einer zusätzlichen Triggerdiode ausgestattet, die einen Rückseitenreflex eines für die Messsignalauskopplung im Messkopf vorgesehenen Strahlteilers ausnutzt.

In einer vorteilhaften Ausführung des flexiblen Laserscanning-Mikroskops ist der Messkopf mechanisch durch einen flexiblen, in beliebigen Stellung arretierbaren Stützarm an einem mobilen Grundgerät abgestützt, der die Übertragungsoptik zwangsweise mitführt, wobei in dem Grundgerät mindestens eine Strahlungsquelle zur Erzeugung gepulster Anregungsstrahlung, eine Optikeinheit zur Synchronisation der gepulsten Anregungsstrahlung und Ausrichtung auf mindestens eine Übertragungsoptik zum Messkopf sowie die Steuereinheit zur Richtungsstabilisierung der Anregungsstrahlung im Messkopf und eine Auswerteeinheit zur Verarbeitung der vom Detektorsystem des Messkopfes übertragenen Signale für die bildgebende Darstellung mit einer Anzeigeeinheit vorhanden sind.

Die Erfindung basiert auf der Überlegung, dass es generell zwei Möglichkeiten gibt, die Genauigkeit einer flexiblen Strahlübertragung bei Spiegel-Gelenkarmen zu erreichen, nämlich durch Verwendung von meist großvolumigen, schweren und kostenintensiven opto-mechanischen Käfigsystemen oder durch Verwendung einer anspruchsvollen Richtungsstabilisierung der Laserstrahlung.

Die Erfindung löst diese divergierenden Anforderungen mit einer technologisch einfachen, kosteneffizienten und kleinvolumigen Richtungsstabilisierung für Laserstrahlung (oder auch inkohärente Strahlung), die zur Abweichungsminimierung einen Detektor, eine Steuereinheit und einen Aktuator aufweist. Abhängig von der detektierten Abweichung in einer konjugierten Zielebene innerhalb eines unbeschränkt flexiblen Messkopfes wird mittels der Steuereinheit (einfache Logik-Schaltung) eine Korrektur der Strahlrichtung des Laserstrahls vorgenommen, bevor dieser in das Beleuchtungs- und Aufnahmesystem (Fokussieroptik) des Messkopfes eingekoppelt wird. Zugleich wird durch diese Strahlstabilisierung auch die Kollinearität der Messstrahlung gesichert, wenn mehrere Anregungsstrahlen verwendet werden.

Dadurch reduzieren sich die an eine mechanische Käfigstruktur eines Spiegel-Gelenkarms gestellten Anforderungen, bezogen auf die benötigte Genauigkeit der Strahlführung, und die resultierenden Volumen- und Gewichtseinsparungen kommen der Flexibilität und Mobilität des mikroskopischen Messsystems (z.B. eines Tomographen) entgegen. Vorteile der Richtungsstabilisierung, wie beispielsweise kompakte und kleinvolumige Bauform und Kosteneffizienz, sind insbesondere bei der Fasereinkopplung in Faserübertragungssystemen zu sehen.

Die erfindungsgemäße Lösung zeichnet sich weiterhin dadurch aus, dass eine zu untersuchende Objektstelle durch ein mikroskopisch-bildgebendes System mit flexiblem Messkopf durch unterschiedliche Messverfahren gleichzeitig untersucht werden kann, wie z.B. mittels hochauflösender Multiphotonen-Fluoreszenzmikroskopie, -SHG-Mikroskopie und/oder CARS-Mikroskopie. Dabei können die unterschiedlichen Anregungsstrahlen von einem oder mehreren Kurzpuls-Lasern über ein und denselben flexiblen optischen Gelenkarm oder einen flexiblen optischen Gelenkarm und eine zusätzliche optische Faser oder über zwei optische Fasern übertragen und mit derselben Einrichtung zur Strahlstabilisierung kontrolliert und reguliert werden.

Mit der Erfindung ist es möglich, ein kompaktes flexibles nichtlineares Laserscanning-Mikroskop zur nicht-invasiven dreidimensionalen Detektion für bildgebende Untersuchungen mit Submikrometer-Auflösung zu realisieren, das eine reproduzierbare Ausleuchtung des Messobjekts bei unbeschränkter Flexibilität des Messkopfes gestattet. Weiterhin lassen sich Strahlengänge von unterschiedlichen Messverfahren, vorzugsweise von Multiphotonen-Fluoreszenzmikroskopie, -SHG-Mikroskopie und/oder CARS-Mikroskopie in einem gemeinsamen flexiblen Messkopf kombinieren und dabei dieselbe reproduzierbare Ausleuchtung und punktgenaue (zellgenaue) Abtastung realisieren.

Die Erfindung soll nachstehend anhand von Ausführungsbeispielen näher erläutert werden. Dazu zeigen die Zeichnungen:
- Fig. 1:: eine Prinzipdarstellung der Erfindung zur Ausrichtung eines Laserstrahls auf einen Zielpunkt in einem Messkopf unter Verwendung eines Spiegel-Gelenkarms als strahlführendes System;
- Fig. 2:: eine Darstellung der Detektionsprinzips für die Strahlabweichung in einer konjugierten Zielebene unter Verwendung einer kreisrunden Photodiode;
- Fig. 3:: eine dreidimensionale Darstellung der Ausführung der Erfindung als mobiles, multivalent einsetzbares Laserscanning-Mikroskop mit frei im Raum positionierbarem Messkopf für Tomographieuntersuchungen an menschlicher Haut;
- Fig. 4:: eine schematische Darstellung einer Ausführung der Erfindung in einem flexiblen Laserscanning-Mikroskop mit zwei Detektionssystemen unter Verwendung eines gemeinsam genutzten Spiegel-Gelenkarms als strahlführendes System zum Messkopf, der für Untersuchungen an einem vertikalen Messobjekt (90°-Stellung), wie z.B. nichtlebenden Substanzen an einem Gemälde, ausgerichtet ist;
- Fig. 5:: eine schematische Darstellung einer Ausführung der Erfindung in einem flexiblen Laserscanning-Mikroskop mit zwei Detektionssystemen unter Verwendung eines Spiegel-Gelenkarms und einer optischen Faser als strahlführende Systeme zum gemeinsamen Messkopf, der zur Untersuchung von lebendem Gewebe von Tieren, z.B. einer Maus, in Normalstellung (0°) ausgerichtet ist,
- Fig. 6:: eine schematische Darstellung einer Ausführung der Erfindung in einem Tomographen mit zwei Detektionssystemen unter Verwendung zweier optischer Fasern als strahlführende Systeme zum gemeinsamen Messkopf, der zur Untersuchung von beliebigen Substanzen, z.B. in flüssiger Lösung in einer Petrischale als inverses Mikroskop (180°-Stellung) ausgerichtet ist.

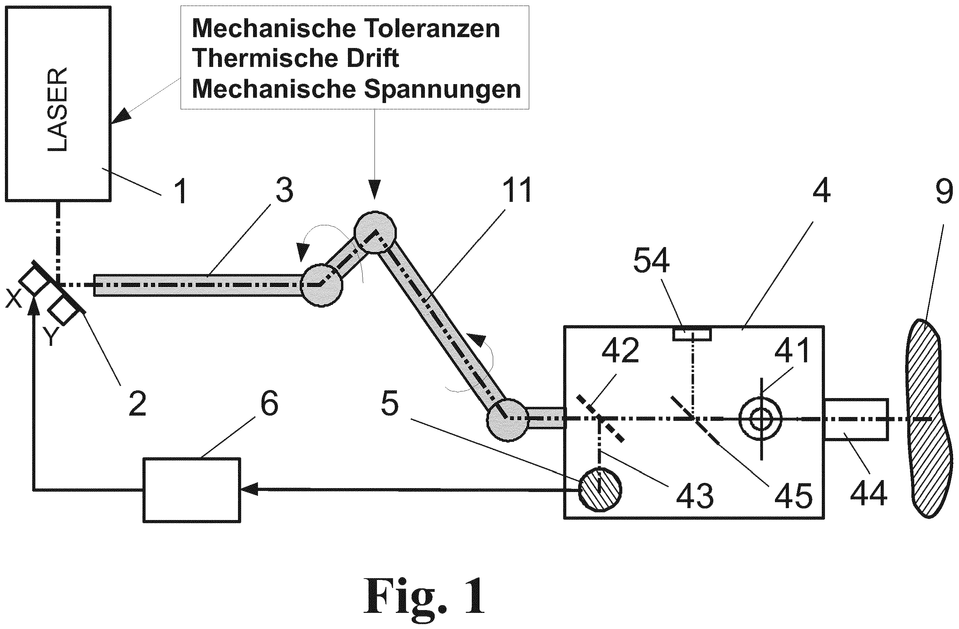

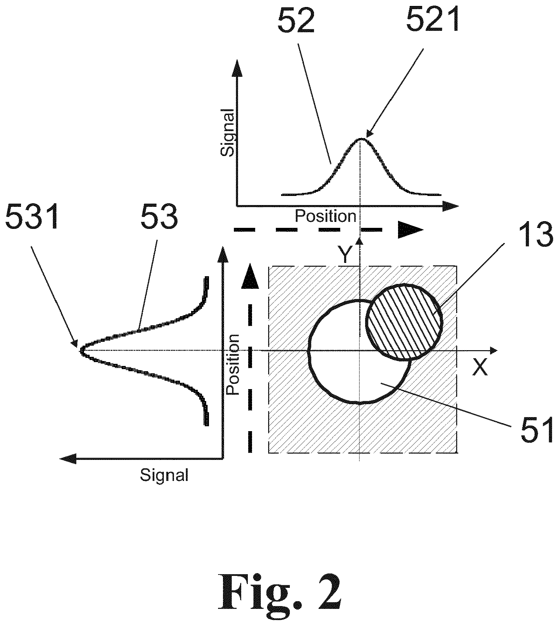

Fig. 1 zeigt den prinzipiellen Aufbau des Systems. Ein Laser 1, vorzugsweise ein Single-Mode-Laser (Transversalmode) mit einem kreisrunden Strahlprofil (oder auch abweichenden Strahlprofil, wie beispielsweise elliptische oder Multimode-Laserstrahlen), sendet einen Laserstrahl 11 auf einen einstellbaren Kippspiegel 2, mit dem der Laserstrahl 11 in eine Übertragungsoptik 3 eingekoppelt und auf eine Zielposition 41 innerhalb eines Messkopfes 4 geleitet wird. Von dem Laserstrahl 11 wird mittels eines im Messkopf 4 angeordneten Strahlteilers 42 ein geringer Anteil (beispielsweise < 10 %) als Teststrahl 43 auf einen ortsauflösenden Photodetektor 5 ausgekoppelt. Die Position des ortsauflösenden Photodetektors 5 ist konjugiert zur einer Zielposition 41 angeordnet, sodass der Photodetektor 5 für Lageabweichungen des Strahlprofils 13 (nur in Fig. 2 gezeigt) des Laserstrahls 11 (bzw. des daraus ausgekoppelten Teststrahls 43) von einer Normal- bzw. Mittenlage empfindlich ist. Das bedeutet, dass der Teststrahl 43 gegenüber dem Laserstrahl 11 eine abweichende optische Achse (beispielsweise unter dem Winkel von 90°) aufweist

Eine Steuereinheit 6 (die als einfache Logik-Einheit realisiert werden kann) wertet das Ausgangssignal des Photodetektor 5 aus und steuert in Abhängigkeit von der Abweichung des Teststrahls 43 (Zentrum bzw. Leistungsschwerpunkt des Strahlprofils 13, wie in Fig. 2 dargestellt) von der Mittenposition des Photodetektors 5 die Winkelausrichtung des einstellbaren Kippspiegels 2 auf eine Maximierung des Photodetektorsignals.

In Fig. 2 ist der ortsauflösende Photodetektor 5 als eine einzelne großflächige kreisrunde Photodiode 51 innerhalb eines schraffierten Quadrats dargestellt. Es gibt jedoch eine Vielzahl von weiteren Möglichkeiten zur Detektion von Lageabweichungen des Strahlprofils 13 des Laserstrahls 11. Diese kann durch ortsauflösend messende Detektoren, wie eine Quadranten-Photodiode, durch zwei orthogonal angeordnete Lateraleffekt-Dioden (engl. position sensitive device - PSD), gekreuzt angeordnete Detektorzeilen (CCD - engl. charge-coupled device oder CMOS - engl. complementary metal oxid semiconductor), eine CCD- oder CMOS-Matrix, wobei die zweidimensionalen Sensorarrays mit kreisrund zusammengeschalteten (Binning) Flächenelementen ausgeführt sind oder im Zusammenwirken mit einer Lichtleitfaser integrierend aufnehmen, realisiert werden. Ferner sind eher seltene Detektorsysteme mit einem bewegten Spalt (engl. scanning slit detector) einsetzbar.

Bei der Änderung der Winkelposition des einstellbaren Kippspiegels 2 (nach Fig. 1) wird das (hier kreisrund angenommene) Strahlprofil 13 des Laserstrahls 11 innerhalb des schraffierten Quadrats ausgelenkt. Bei einer anteiligen Überlagerung der Flächen des Strahlprofils 13 und des Photodetektors 5, die vorteilhaft entsprechend dem kreisrunden Strahlprofil 13 als kreisrunde Photodiode 51 ausgebildet sein sollte, wird ein Ausgangssignal erzeugt, das auf dem durch die einfallende Laserstrahlung erzeugten Photostrom des Photodetektors 5 basiert.

Ziel der Anordnung gemäß Fig. 1 ist eine permanente Zentrierung des Strahlprofils 13 im Zentrum des Photodetektor 5. Die Position des Strahlprofils 13 ist hierbei durch die Koordinaten X und Y innerhalb eines kartesischen Koordinatensystems definiert, das der idealen Position des Laserstrahls 11 in der Zielposition 41 des Messkopfes 4 zugeordnet ist. Dasselbe Koordinatensystem ist der konjugierten Position, in der der Photodetektor 5 angeordnet ist, zugeordnet und stellt die Basis der Erfassung der Strahlabweichung dar. Der Ursprung des kartesischen Koordinatensystems der Zielposition 41 in Fig. 1 korreliert mit dem Zentrum der kreisrunden Photodiode 51, da diese in einer konjugierten Ebene zur Zielposition 41 angeordnet ist.

Das Ausgangssignal des Photodetektors 5 entsteht durch eine positionsabhängige Überlagerung bzw. Faltung der Empfindlichkeitsverteilung der kreisrunden Photodiode 51 mit der Intensitätsfunktion des Strahlprofils 13, wie sie in Fig. 2 beispielsweise als annähernde Gaußverteilung für die Signalkomponente 52 in der X-Koordinate und als qualitativ gleichartige Signalkomponente 53 in der Y-Koordinate innerhalb des schraffierten Quadrats (angenommenes Koordinatensystem) dargestellt ist. Infolge der Aufteilung des Ausgangssignals in X- und Y-Komponenten ist die Position des Strahlprofils 13 mittels der Winkelauslenkung des einstellbaren Kippspiegels 2 veränderbar und schrittweise (z.B. zunächst für die X- und dann für die Y-Koordinate auf die Maximierung des Photodiodensignals) einstellbar.

Eine Auslenkung des Strahlprofils 13 in X-Richtung eines kartesischen Koordinatensystems erzeugt eine positionsabhängige Signalkomponente 52 mit einem globalen Maximum 521. Die Amplitude der Signalkomponente 52 ist von der Überlagerung des (kreisrunden) Strahlprofils 13 und der kreisrunden Photodiode 51, oder genauer gesagt, von der Faltung der Intensitätsfunktion des Laserstrahls 11 mit der Empfindlichkeitsfunktion der Photodiode 51 abhängig. Der zu dem Maximum 521 gehörige Auslenkungswinkel des Laserstrahls 11 repräsentiert die gesuchte Koordinate in X-Richtung. Damit ist die gesuchte Korrektur-Position der X-Achse des Kippspiegels 2 ermittelt und ausgerichtet. Ausgehend von dieser X-Koordinate führt eine Auslenkung in orthogonaler Richtung (der Y-Achse des Koordinatensystems) zu einer zweiten Signalkomponente 53 der kreisrunden Photodiode 51, deren Maximum 531 mit der gesuchten Koordinate in Y-Richtung korreliert. Die gesuchte Korrektur-Position der Y-Achse des Kippspiegels 2 ist auf diese Weise ermittelt und der Kippspiegel 2 kann entsprechend ausgerichtet werden. Die Amplitude der Signalkomponente 53 ist infolge der ersten Nachführbewegung durch die vergrößerte Überlagerung von Strahlprofil 13 und Fläche der Photodiode 51 gegenüber Signalkomponente 52 vergrößert bzw. maximiert.

Für ein ideales kreisrundes gaußförmiges Strahlprofil 13 und eine ideal kreisrunde Photodiode 51 genügt ein einmaliges Ermitteln der Koordinaten. Hierbei korreliert nach dem Anfahren der Koordinaten in X- und Y-Richtung der Ursprung des kartesischen Koordinatensystems der Zielposition 41 mit dem Zentrum bzw. Schwerpunkt des Strahlprofils 13.

Für ein nicht ideales (abweichend von kreisrund gaußförmig) Strahlprofil 13 bzw. einen nicht kreisrunden Photodetektor 51 muss der Ablauf der Koordinatenfindung in X- und Y-Richtung mehrmals wiederholt werden. Das Ergebnis der ersten Ausrichtung kann dann erneut im Teststrahl 43 überprüft und gegebenenfalls der Kippspiegel 2 nochmals nachgestellt werden.

Durch mehrfache Anwendung des Zentriervorgangs können demnach neben kreisrunden (Single- Mode) Strahlprofilen auch sogenannte Multimode- bzw. auch nicht-symmetrische Strahlprofile 13 auf die Zielposition 41 zentriert werden (iterative Annäherung). Ausgerichtet wird in diesem Fall nicht das Zentrum eines idealen kreisrunden Strahlprofils 13, sondern der Intensitätsschwerpunkt des Strahlprofils 13. Mit Hilfe der Information beider Signalkomponenten 52 und 53 der kreisrunden Photodiode 51 in den Koordinaten X und Y wird mittels der Steuereinheit 6 der einstellbare Kippspiegel 2 so ausgerichtet, dass das Signal der Photodiode 51 größer wird und somit das Strahlprofil 13 (bzw. dessen Intensitätsschwerpunkt) auf das Zentrum der Zielebene 41 verschoben wird.

Für maximale Genauigkeiten dieses Regelungsprinzips müssen die Signalkomponenten 52 und 53 ein plateaufreies Maximum 521 bzw. 531 aufweisen. Dies kann durch das Abgleichen des Durchmessers der kreisrunden Photodiode 51 an den Durchmesser des Strahlprofils 13 erfolgen, wobei der Durchmesser der Photodiode 51 doppelt so groß wie der Strahlradius bei 1/e2 der Intensität des Laserstrahls 1 sein sollte. Eine weitere Möglichkeit, das Maximum 521 bzw. 531 der Signalkomponente 52 bzw. 53 so genau wie möglich zu ermitteln, ist eine nachfolgende Signalverarbeitung (z. B. gaußförmige Anpassung oder Tiefpassfilterung). Dies erlaubt den Betrieb des Systems auch mit langkohärenten Laser-Strahlungsquellen, bei denen das Signal durch Mehrfachspiegelung an Strahlteilern Interferenzen aufweist.

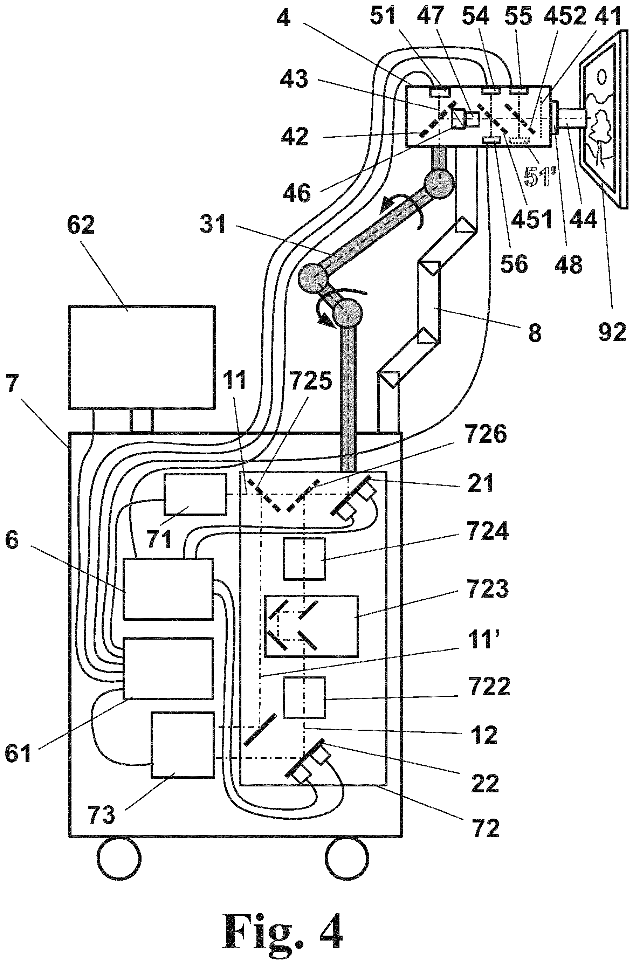

Der Dynamikbereich der Signalkomponenten 52 und 53 kann durch Verwendung logarithmischer Verstärker (nicht gezeichnet) erhöht werden, was die Funktion der Richtungsstabilisierung auch bei Lasern mit variabler Ausgangsleistung garantiert. Für die Erzeugung des Teststrahls 43 kann entweder die Reflexion (wie in Fig. 1 gezeigt) oder die Transmission des Laserstrahls 11 am Strahlteiler 42 (wie in Fig. 4 gezeichnet) genutzt werden je nachdem, in welcher Richtung der Laserstrahl 11 im Messkopf 4 weiter geführt werden soll.

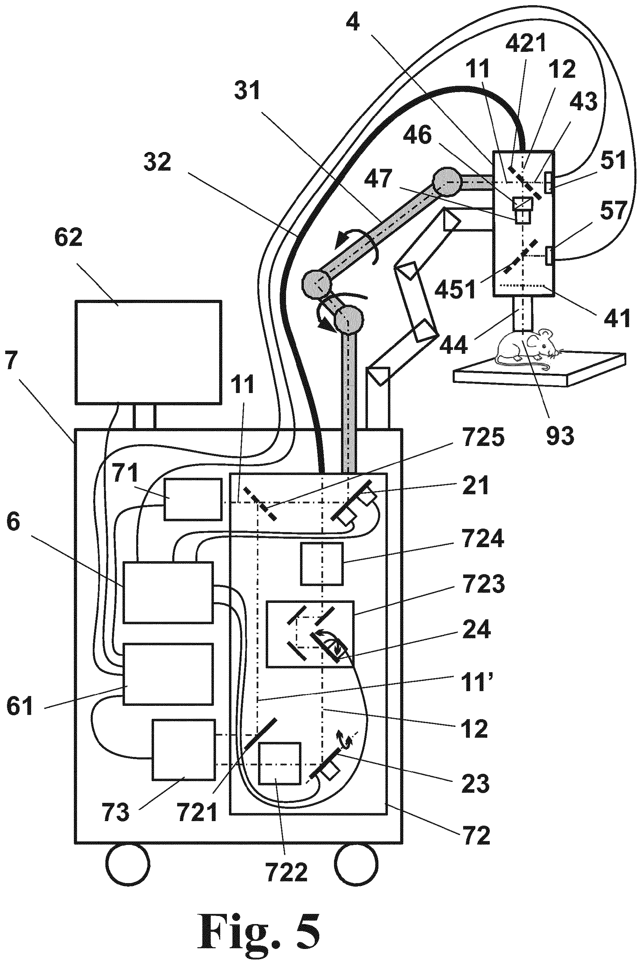

Bei der Verwendung von Faserübertragungsoptiken (wie in Fig. 5 vorhanden) wird die Einkopplung des Laserstrahls 11 und demnach die Transmission des Systems durch thermische Drift und mechanische Spannungen innerhalb der Optikeinheit 72 beeinflusst. Werden Monomode-Fasern verwendet, deren Kerndurchmesser sehr klein sind, kann die Transmission innerhalb von nur wenigen Minuten um mehrere 10 Prozent abfallen. Hierbei kann es neben einer unzureichenden Laserleistung im Messkopf 4 zu einer Zerstörung des Faserübertragungssystems durch thermische Wechselwirkungen innerhalb der Faser kommen, die unbedingt vermieden werden müssen.

Das Wirkprinzip der Korrektur des Laserstrahls 11 bzw. dessen Zentrierung in den Kern einer Faserübertragungsoptik erfolgt analog zur Darstellung von Fig. 1, wobei die Funktion der in Fig. 2 beschriebenen kreisrunden Photodiode 51 durch den kreisrunden Kerndurchmesser der als Faser ausgebildeten Übertragungsoptik 3 und einen dahinter geschalteten, alles übertragene Licht integrierenden Photodetektor 5 repräsentiert sein kann. Die Lichtleitfaser 32 bildet dabei zusammen mit einem dahinter positionierten Photodetektor 5 beliebiger Form eine für die Regelung mit der kreisrunden Photodiode 51 gemäß Fig. 1 und Fig. 2 beschriebene Funktion äquivalent nach.

### Ausführungsbeispiel 1

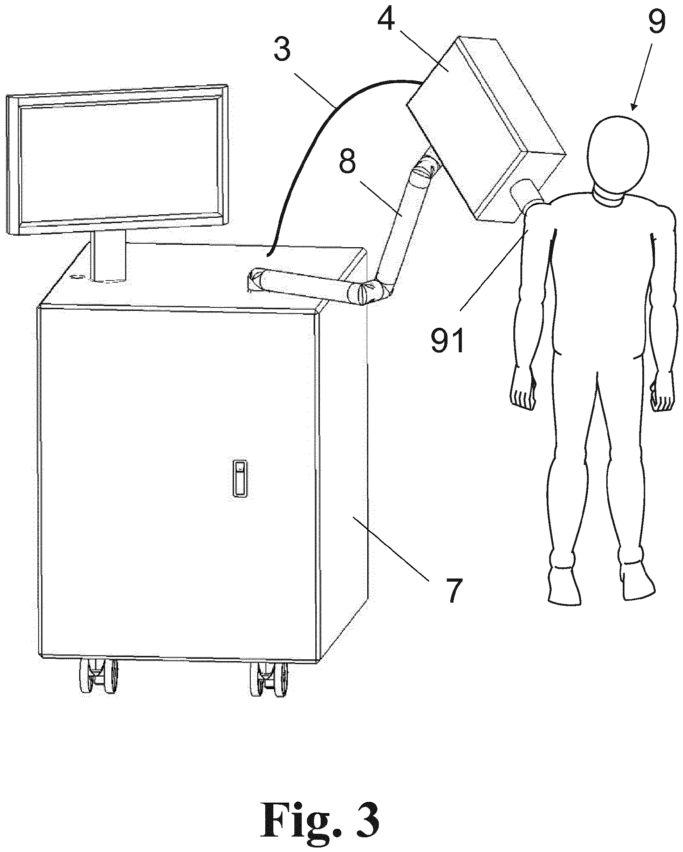

Wie in Fig. 3 dargestellt, besteht das Messsystem als mobiles, flexibles nichtlineares Scanning-Mikroskop aus einem mobilen Grundgerät 7 auf Rädern zur frei wählbaren Platzierung. An diesem Grundgerät ist über einen Stützarm 8 ein frei im Raum beweglicher Messkopf 4 befestigt. Zusätzlich sind zur optischen Strahlführung der für das bildgebende Laserscanning benötigten Laserstrahlung zwei unterschiedliche optische Übertragungsoptiken 3, ein Spiegel-Gelenkarm 31 und eine optische Faser 32, zwischen dem Grundgerät 7 und dem Messkopf 4 angebracht. Diese sind zur Übertragung der Anregungsstrahlung für unterschiedliche Detektionsverfahren, z.B. - ohne Beschränkung der Allgemeinheit - für Multiphotonen- und CARS-Tomographie, vorgesehen, wobei die unterschiedlichen Anregungsstrahlen im Messkopf 4 kollinear überlagert werden. Dazu ist im Messkopf 4 das in Fig. 1 prinzipiell gezeigte System der Strahlstabilisierung mit positionsempfindlichem Photodetektor 5, Steuereinheit 6 und steuerbarem Kippspiegel 2 eingesetzt. Dadurch ist die in Fig. 3 gezeigte freie Ausrichtung des Messkopfes 4 auf eine beliebig im Raum liegenden Abschnitt menschlicher Haut 91 als Messobjekt 9 (Mensch) möglich, ohne dass sich irgendwelche Änderungen (Verschlechterungen) der Anregungsbedingungen infolge der vielfältigen Verschwenkungsmöglichkeiten des Messkopfes 4 ergeben.

Weitere verschiedene technische Ausgestaltungen des erfindungsgemäßen Scanning-Mikroskops inklusive einiger der vielfältigen Anwendungsmöglichkeiten werden in den nachfolgenden Beispielen im Detail erläutert.

### Ausführungsbeispiel 2

Das erfindungsgemäße Mikroskop von Fig. 4 besteht, wie zu Fig. 3 oben beschrieben, aus einem mobilen Grundgerät 7 auf Rädern zur frei wählbaren Platzierung.

Für die Multiphotonenbildgebung ist im Grundgerät 7 ein Kurzpuls-Lasersystem 71 (5 fs-500 ps; mit gaußförmigem Strahlprofil, z.B. ein Titan:Saphir-Laser) sowie eine optische Einheit 72 vorhanden, in der der steuerbare Kippspiegel 2 als zweiachsiger Kippspiegel 21 den vom Kurzpuls-Lasersystem 71 kommenden Laserstahl 11 in eine flexible Übertragungsoptik 3 in Form eines Spiegel-Gelenkarms 31 eingekoppelt. Der Laserstrahl 11 wird in diesem ersten Beispiel über den flexiblen Spiegel-Gelenkarm 31 zum optischen Messkopf 4 geleitet. Ein beweglicher und in jeder Stellung arretierbarer mehrgliedriger Stützarm 8 ermöglicht die freie Positionierung des optischen Messkopfes 4, sodass der Spiegel-Gelenkarm 31 entsprechend zwangsweise mitgeführt wird. Der Messkopf 4 ist somit mit seiner Fokussieroptik 44 in beliebiger Ausrichtung auf das Messobjekt 9, das im Falle in einer 90°-Stellung auf ein Messobjekt 9 mit senkrechter Oberfläche, in diesem Beispiel ein hängendes Gemälde 92, dessen Zusammensetzung (Material oder Schichtaufbau) zu untersuchen ist, positionierbar. Dabei wird aufgrund der ständigen Kontrolle und Nachführung der Achsrichtung des Laserstrahls 11 die Zielebene 41 stets gleichbleibend ausgeleuchtet, sodass Messfehler, die aus schwankender Anregungsstrahlung resultieren, vermieden werden.

Die für die CARS-Bildgebung (zusätzlich zur MPT-Bildgebung) erforderliche zweite Strahlquelle mit einer vom Kurzpuls-Laser 71 abweichenden Wellenlänge wird aus dem Kurzpuls-Laser 71 erzeugt, indem über einen Strahlteiler 725 ein ca. hälftiger Strahlungsanteil aus dem Laserstrahl 11 ausgekoppelt und als Laserstrahl 11' einem Frequenzshifter 73 zugeführt wird. Der daraus abgegebene Laserstrahl 12 mit abweichender Wellenlänge wird ebenfalls in die Optikeinheit 72 eingekoppelt, wobei der Laserstrahl 12 über einen zweiten zweiachsig steuerbaren Kippspiegel 22 (mit gleicher Funktion wie zweiachsige Kippspiegel 21) zusätzlich durch ein Fernrohrsystem 722 mit variablem Linsenabstand und eine variable optische Verzögerungsstrecke 723 geführt wird und nach Durchlaufen einer justierbaren Spiegelanordnung 724 und eines dichroitischen Spiegels 726 mit dem Laserstrahl 11 des Kurzpuls-Lasers 71 kollinear (räumliche Überlagerung beider Strahlanteile entlang derselben Achse) ausgerichtet ist. Beide Laserstrahlen 11 und 12 werden nach dem dichroitischen Spiegel 726 kollinear in den Spiegel-Gelenkarm 31 eingekoppelt und zum Messkopf 4 übertragen. Die Achsenübereinstimmung der beiden Laserstrahlen 11 und 12 wird dann im Messkopf 4 permanent überwacht, indem die mittige Ausleuchtung der positionsempfindlichen Photodiode 51 in dem aus dem Strahlweg zur Fokussieroptik 44 über den Strahlteiler 42 ausgekoppelten Teststrahl 43 überprüft und mittels der Steuereinheit 6 die festgestellte Abweichung über die zweiachsig steuerbaren Kippspiegel 21 und 22 der Optikeinheit 72 für jeden der Laserstrahlen 11 und 12 korrigiert wird. Dadurch wird bei jeder beliebigen Ausrichtung des Spiegel-Gelenkarms 31 gewährleistet, dass die Eintrittspupille der Fokussieroptik 44 (Zielebene 41) homogen ausgeleuchtet ist. Anstelle der Eintrittspupille der Fokussieroptik 44 kann auch eine Aperturbegrenzung eines anderen im Messkopf 4 verwendeten optischen Elements verwendet werden.

Die zeitliche Überlagerung der einzelnen Laserimpulse der beiden Laserstrahlen 11 und 12 wird durch manuelles oder motorisiertes Verstellen der variablen optischen Verzögerungsstrecke 723 realisiert. Die nach der Fokussieroptik 44 durch Dispersion voneinander getrennten Fokuspositionen der in der Wellenlänge unterschiedlichen Laserstrahlen 11 und 12 können durch das Fernrohrsystem 722 innerhalb des Messobjekts 9 (im vorliegenden Beispiel eines Gemäldes 92) zur Überlagerung gebracht werden.

Innerhalb des Messkopfes 4 wird der Hauptanteil der Laserstrahlung der beiden Laserstrahlen 11 und 12 über den Strahlteiler 42 in eine für einen zweidimensionalen Scan vorgesehene Scaneinheit 46 geführt. Nach der Scaneinheit 46 wird die Laserstrahlung über ein Fernrohrsystem 47 in die Eintrittspupille der Fokussieroptik 44 geführt und mit der Fokussieroptik 44 im zu untersuchenden Gemälde 92 fokussiert.

Die Scaneinheit 46 erlaubt eine zweidimensionale Auslenkung der Laserstrahlen 11 und 12 innerhalb der Fokalebene der Fokussieroptik 44, sodass ein rasterförmiges zweidimensionales Scannen bzw. Anregen des Gemäldes 92 erfolgen kann.

Die tomographische dreidimensionale Darstellung des Messobjekts 9 (als Stapelaufnahme über verschiedene Schichten des Gemäldes 92) wird durch das systematische Verstellen der Fokussieroptik 44 über einen Z-Achsen-Positionierer 48 ermöglicht.

Die in der Fokalebene der Fokussieroptik 44 im Gemälde 92 jeweils von den unterschiedlichen Anregungsstrahlen (Laserstrahlen 11 und 12 mit verschiedener Wellenlänge und Pulsfolge) generierten Signale werden durch die Fokussieroptik 44 des Messkopfes 4 integrierend aufgenommen und mittels zweier dichroitischer Strahlteiler 451 und 452 zur Auskopplung der Messsignale, beispielsweise Autofluoreszenz- und SHG-Signale, von der reflektierten Anregungsstrahlung getrennt und auf zwei Messdetektoren, MP-Detektor 54 und CARS-Detektor 55, gelenkt. Die Auswertung der MP- und CARS-Signale erfolgt im Grundgerät 7 in einer Auswerteeinheit 61 und kann auf einer Anzeigeeinheit 62 als mehrdimensionale Grauwertverteilung bildlich dargestellt werden.

Für eine auf zeitkorrelierter Einzelphotonenzählung (engl. Time Correlated Single Photon Counting - TCSPC) basierte Bildaufnahme mit dem Ziel, Atome und Moleküle und deren Umgebung durch Messung der Abklingzeiten der Atom- und Molekülschwingungen zu spezifizieren, können die Messdetektoren 54 und 55 durch Detektoren hoher zeitlicher Auflösung ersetzt und deren Signale in der Auswerteeinheit 61 verarbeitet werden. Für die bei TCSPC-Messungen erforderliche Triggerung ist eine Triggerdiode 56 vorgesehen, die einen parasitären Rückseitenreflex eines der Strahlteiler 451 oder 452 ausnutzt. In diesem Beispiel wird der Rückseitenreflex des Strahlteilers 451 verwendet.

Optional kann auch für die positionsempfindliche Photodiode 51, mit deren Ausgangssignal die Steuereinheit 6 über die zweiachsigen Kippspiegel 21 und 22 die stabile Lage der Laserstrahlen 11 und 12 bezüglich der im Messkopf 4 definierten Zielebene 41 steuert, ein parasitärer Rückseitenreflex eines der Strahlteiler 451 oder 452 ausgenutzt werden. Dazu ist in Fig. 4 diese zur Photodiode 51 alternative Position gestrichelt eingezeichnet und mit Photodiode 51' bezeichnet, wobei der parasitäre Rückseitenreflex vom Strahlteiler 452 ausgenutzt wird. Diese alternative Photodiode 51' kann, da sie näher an der Zielebene 41 (Eintrittspupille der Fokussieroptik 44) liegt, bevorzugt werden und erübrigt dann die im Teststrahl 43 angeordnete Photodiode 51. Der Strahlteiler 42 kann in diesem Fall durch einen Vollspiegel (nicht gezeichnet) ersetzt werden

Mit dieser laserstrahlstabilisierten Anregung des zu vermessenden Gemäldes 92 wird die Richtungsstabilität um den Faktor 10-20 verbessert. Eine derartige Verbesserung sichert eine homogene Ausleuchtung des Bildfeldes in allen Positionen des Messkopfs 4 und mithin reproduzierbare, vergleichbare Messergebnisse.

### Ausführungsbeispiel 3

Fig. 5 zeigt das Messsystem unter Verwendung eines Spiegel-Gelenkarms 31 und einer optischen Faser 32 als zwei getrennte strahlführende optische Übertragungssysteme 3.

Der Kurzpuls-Laser 71 wird in gleicher Weise - wie im zweiten Beispiel - mit der optischen Einheit 72 über den zweiachsigen Kippspiegel 21 in den flexiblen Spiegel-Gelenkarm 31 eingekoppelt und zum optischen Messkopf 4 geleitet.

Der für die Erzeugung der zusätzlichen CARS-Bildgebung verwendete Frequenzshifter 73 wird mittels des vom Stahlteiler 725 ausgekoppelten Laserstrahls 11' über einen Umlenkspiegel 721 mit der vom Kurzpuls-Laser 71 erzeugten Ausgangs-Wellenlänge versorgt und sendet einen Laserstrahl 12 mit vom Kurzpuls-Laser 71 abweichender Wellenlänge aus. Der Laserstrahl 12 wird dann - abweichend vom vorigen Beispiel -, nachdem er zunächst das Fernrohrsystem 722 durchlaufen hat, mittels eines ersten einachsigen Kippspiegels 23 und eines dazu orthogonal angeordneten zweiten einachsigen Kippspiegels 24 vor der optischen Verzögerungsstrecke 723 in zwei Koordinatenrichtungen getrennt ausgerichtet. Ansonsten durchläuft er, wie zu Fig. 4 beschrieben, die Verzögerungsstrecke 723 und die Spiegelanordnung 724. Danach passiert der Laserstrahl 12 - ebenfalls abweichend vom Aufbau in Fig. 4 - keinen dichroitischen Strahlteiler 726 zum Zusammenführen mit dem Laserstrahl 11, sondern er wird separat in eine Lichtleitfaser 32 eingekoppelt und über diese zum Messkopf 4 geführt.

Im Messkopf 4 wird der Laserstrahl 12 über einen geeigneten dichroitischen Teiler 421, der zugleich für den Laserstrahl 11 die Umlenkfunktion sowie die Teilerfunktion zur Auskopplung des Teststrahls 43 auf die Photodiode 51 übernimmt, mit dem aus dem Spiegel-Gelenkarm 31 zugeleiteten Laserstrahl 11 kollinear überlagert und in das Gewebe einer Maus 93 (als konkretes Messobjekt 9) fokussiert.

Die weitere Erfassung und Verarbeitung der aus dem Gewebe einer Maus 93 zurück kommenden Signale erfolgt in diesem Beispiel ausgekoppelt über den Strahlteiler 451 mittels eines gemeinsamen Detektors 57 für die erzeugten MP- und CARS-Signale, wobei durch das Blockieren eines der Laserstrahlen 11 oder 12 oder alternativ durch Änderung der Phasenlage (zeitlicher Bezug) beider Laserstrahlen 11 und 12 zueinander mittels der Verzögerungsgruppe 723 die CARS-Signale von den MP-Signalen diskriminiert werden können, wodurch der Detektor 57 nur MP-Signale empfängt. Außerdem können durch phasenrichtige Anregung beider Laserstrahlen 11 und 12 die CARS- und MP-Signale des Gewebes der Maus 93 zusammen (überlagert) detektiert werden.

### Ausführungsbeispiel 4

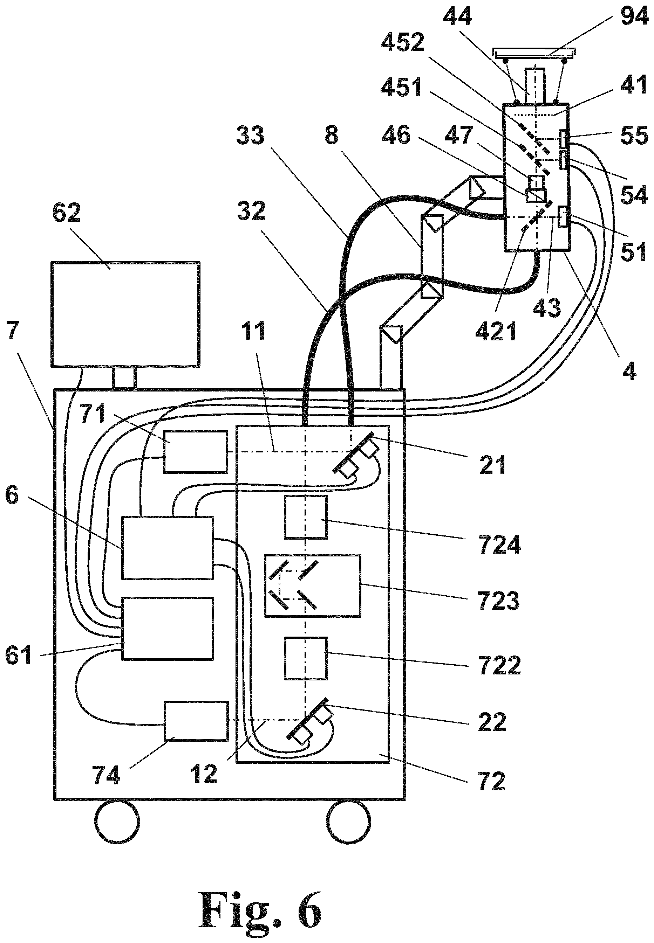

Eine weitere vorteilhafte Ausgestaltung des Laserscanning-Mikroskops ist in Fig. 6 dargestellt, wobei zwei optische Fasern 32 und 33 als strahlführende optische Übertragungssysteme 3 verwendet werden.

Die beiden Laserstrahlen 11 und 12 mit unterschiedlicher Wellenlänge werden in der Optikeinheit 72 wie im dritten Beispiel geführt, ausgerichtet und aufeinander abgestimmt, ohne miteinander überlagert zu werden. Der Laserstrahl 12 wird jedoch im Unterschied zu den vorigen Beispielen in einer zweiten Laserquelle 74 erzeugt und nach einer etwas aufwändigeren Phasensynchronisation in der Verzögerungsgruppe 723 und anschließende Spiegelanordnung 724 in die Lichtleitfaser 32 (wie im dritten Beispiel gemäß Fig. 5) eingekoppelt. Der Laserstrahl 11 ist in diesem Fall in die zweite Faser, vorzugsweise eine Monomodefaser 33, eingekoppelt. Beide Fasern 32 und 33 werden dann im Messkopf 4, in analoger Art wie im dritten Beispiel, mittels eines dichroitischen Teilers 421 kollinear überlagert. Die stabilisierte kollineare Ausrichtung der Laserstrahlen 11 und 12 sowie die Erfassung der damit angeregten Signale geschehen in gleicher Weise, wie im zweiten Ausführungsbeispiel beschrieben.

Als Messobjekt 9 kann eine mit flüssigen oder festen Substanzen befüllte Petrischale 94 am Messkopf 4 starr (im einfachsten Fall z.B. mit doppelseitigem Klebeband) fixiert sein oder mit einem Positioniertisch (z.B. zwei- oder dreiachsiger Kreuztisch, nicht gezeigt) beweglich angekoppelt werden. Dies ermöglicht eine mikroskopische Bildgebung bei sehr geringem technischen Aufwand. Anstelle einer Petrischale 94 können auch Mikrotiterplatten in gleicher Weise auf deren Substanzen untersucht werden. Die in Fig. 6 stilisiert gezeigte inverse Mikroskopanordnung erlaubt somit die Untersuchung von beliebigen Proben, wie beispielsweise in Nährlösungen eingebettete Proben, feste pulverisierte oder granulierte Stoffe oder auch Festkörper (oberflächennahe Bereiche).

Die Vorteile des beschriebenen MPT-Systems mit CARS-Detektion liegen gegenüber konventionellen und unflexiblen Systemen in dem durch den optischen Gelenkarm 31 und/oder Lichtleitfasern 32, 33 im Raum frei positionierbarem Messkopf 4 mit geringem Gewicht und dem mobilen Grundgerät 7. Eine solche Anordnung erlaubt es dem Bediener, in kurzer Zeit hochauflösende kombinierte Messungen (Zeit- und Kostenersparnis) reproduzierbar durchführen zu können und dabei über eine mikroskopische Messanordnung zu verfügen, die für beliebig ausgerichtete Objektoberflächen völlig gleichwertige (kompatible) und ohne Messobjektbewegung (z.B. Patientenumlagerungen) oder Probenentnahmen vom Messobjekt 9 eine Vielzahl von Messungen an verschiedenen Teilen des Messobjekts 9 (insbesondere des menschlichen oder tierischen Körpers) durchführen zu können.

Des Weiteren ermöglicht die flexible Positionierung des Messkopfes 4 eine sogenannte inverse Bildgebung, wie sie in der herkömmlichen Mikroskopie gern genutzt wird.

Im Unterschied zu herkömmlichen Mikroskopsystemen, die nur sowohl als aufrechtes Mikroskop (0°) als auch als inverses Mikroskop (180°) verfügbar sind, erlaubt die vorliegende Erfindung mit dem unbeschränkt beweglichen Messkopfes 4 auch die Anordnung in 90°-Stellung und beliebigen Abweichung davon. Dadurch sind ebenfalls Messungen an stehenden Objekten möglich, wie z.B. Untersuchungen von Schimmelkulturen an Wänden, von Pflanzenteilen innerhalb einer Pflanzung, Materialanalyse (siehe Fig. 4), Tieruntersuchungen (siehe Fig. 5) und Hautuntersuchungen an Menschen (Fig. 3) sowie kriminalistische Untersuchungen verschiedenster Art, wie auch die Nutzung als inverses Laserscanning-Mikroskop zur Untersuchung von beliebigen flüssigen oder festen Substanzen (Fig. 6).

Mit einem entsprechend angepassten Aufsatz (nicht gezeichnet) am Messkopf 4 ist es ferner möglich, einen Positioniertisch (Kreuztisch) am Messkopf 4 zu verwenden, um eine herkömmliche Mikroskopperformance anzubieten, die aufgrund der Mobilität des Scanning-Mikroskops (Grundgerät 7) an jedem beliebigen Ort hochauflösende Untersuchungen über makroskopische Probenbereiche gestattet.

Die Vorteile der Kopplung von bildgebenden Diagnosesystemen der Multiphotonen-Fluoreszenzmikroskopie, -SHG-Mikroskopie und/oder CARS-Mikroskopie gegenüber jeweils separaten Diagnose-Systemen liegen in der Kombination der Verfahren, der Multiphotonen-, -SHG- und CARS-Anregung und -Bildgebung in einem kompakten mobilen Grundgerät 7 mit flexiblem Messkopf 4. Die vorgeschlagene Anordnung mit flexiblem Gelenkarm 31 bzw. optischer Faser 32, 33 erlaubt eine freie Positionierung eines Messkopfes 4 ohne Beschränkungen der Auflösung und ohne Signalverfälschungen infolge von Fehljustage der Anregungsstrahlung, indem letztere auf einfache und robuste Weise zuverlässig erfasst und korrigiert wird und somit eine fehlerminimierte Bildgebung und Auswertung gewährleistet ist.

### Bezugszeichenliste

- 1: Laser
- 11: Laserstrahl (Anregungsstrahl)
- 12: Laserstrahl (zweiter Anregungsstrahl)
- 13: Strahlprofil

- 2: (steuerbarer) Kippspiegel
- 21, 22: zweiachsiger Kippspiegel
- 23, 24: einachsiger Kippspiegel

- 3: Übertragungsoptik
- 31: Spiegel-Gelenkoptik
- 32: Lichtleitfaser
- 33: Monomodefaser

- 4: Messkopf
- 41: Zielposition
- 42: Strahlteiler
- 421: dichroitischer Teiler
- 43: Teststrahl
- 44: Fokussieroptik
- 45: Signalauskoppel-Teiler
- 451: dichroitischer Strahlteiler
- 452: dichroitischer Strahlteiler
- 46: Scaneinheit
- 47: Fernrohrsystem
- 48: Z-Achsen-Positionierung

- 5: (positionsempfindlicher) Photodetektor
- 51: (kreisrunde) Photodiode
- 52, 53: Signalkomponente (in X-, Y-Richtung)
- 521,531: Maximum
- 54: (MP-)Detektor
- 55: (CARS-) Detektor
- 56: Triggerdiode
- 57: gemeinsamer Detektor

- 6: Steuereinheit
- 61: Auswerteeinheit
- 62: Anzeigeeinheit

- 7: (mobiles) Grundgerät
- 71: Kurzpuls-Laser
- 72: Optikeinheit
- 721: Umlenkspiegel
- 722: teleskopisches System
- 723: Verzögerungsgruppe
- 724: Spiegelanordnung
- 725: Strahlteiler
- 726: dichroitischer Spiegel
- 73: Frequenzshifter
- 74: zweite Laserquelle

- 8: Stützarm

- 9: Messobjekt
- 91: menschliche Haut
- 92: Gemälde
- 93: Gewebe einer Maus
- 94: Petrischale (mit flüssiger Substanz)

## Patentansprüche

1. Flexibles nichtlineares Laserscanning-Mikroskop zur nicht-invasiven dreidimensionalen Detektion, enthaltend mindestens eine Strahlungsquelle, die wenigstens einen Anregungsstrahl gepulster Laserstrahlung zur Auslösung einer von Atomen und Molekülen emittierten Sekundärstrahlung erzeugt, eine flexible Übertragungsoptik zur Übertragung der Strahlung zu einem Messkopf mit einer Fokussieroptik, mit der die Strahlung in ein Messobjekt fokussiert und die emittierte Sekundärstrahlung zurückgeführt und auf mindestens ein Detektorsystem gerichtet ist, wobei der Messkopf (4) frei im Raum schwenk- und drehbar und flexibel positionierbar mit der mindestens einen Strahlungsquelle (1; 71; 74) verbunden ist, sodass aufrechte, inverse sowie Mikroskopie unter beliebigen Raumwinkeln ausführbar ist, **dadurch gekennzeichnet, dass**
- mindestens ein steuerbarer Kippspiegel (2) zur Umlenkung und Ausrichtung des wenigstens einen Anregungsstrahls (11; 12) gepulster Laserstrahlung der mindestens einen Strahlungsquelle derart angeordnet ist, um den wenigstens einen Anregungsstrahl (11; 12) durch die Übertragungsoptik (3) hindurch so in den Messkopf (4) zu richten, dass der wenigstens eine Anregungsstrahl (11; 12) in jeder beliebigen Position des Messkopfes (4) konzentrisch zu einem aperturbegrenzten optischen Element des Messkopfes (44) angeordnet ist,
- ein Strahlteiler (42) zur Auskopplung eines geringen Anteils aus dem wenigstens einen Anregungsstrahl (11; 12) im Messkopf (4) vor der Fokussieroptik (44) angeordnet ist, um einen Teststrahl (43) zu erzeugen,
- eine Photodiode (5) in einer zu einer Zielebene (41) konjugierten Ebene im Teststrahl (43) angeordnet ist, wobei eine Mittenausrichtung des Teststrahls (43) auf der Photodiode (5) der Normalausrichtung des Anregungsstrahls (11, 12) in der Zielebene entspricht, und
- durch Auslenkung des Anregungsstrahls in X- und Y-Richtung nacheinander jeweils positionsabhängige X- und Y-Signalkomponenten (52, 53) erzeugt werden, wobei das jeweilige Maximum (521, 531) der positionsabhängigen X- und Y-Signalkomponenten der Mittenausrichtung des Teststrahls (43) auf der Photodiode (5) entspricht,
- eine Steuereinheit (6) zur Ansteuerung des steuerbaren Kippspiegels (2) vorhanden ist, die mit Hilfe der positionsabhängigen X- und Y-Signalkomponenten den steuerbaren Kippspiegel so ausrichtet, dass der Teststrahl mittig auf die Photodiode gerichtet wird, sodass eine Richtungsstabilisierung des wenigstens einen Anregungsstrahls unabhängig von der Größe der positionsbedingten Beeinträchtigung der Übertragungsoptik (3) erreicht wird.

2. Laserscanning-Mikroskop nach Anspruch 1, **dadurch gekennzeichnet, dass** der mindestens eine steuerbare Kippspiegel (2) mittels mindestens eines steuerbaren Spiegelhalters auf Basis eines Antriebsprinzips aus der Gruppe von kapazitiven, induktiven oder Piezo-Stellern, Schrittmotoren oder Gleichstrommotoren angetrieben ist.

3. Laserscanning-Mikroskop nach Anspruch 1, **dadurch gekennzeichnet, dass** der mindestens eine steuerbare Kippspiegel (2) mittels eines zweiachsigen Spiegelhalters (21; 22) oder zweier einachsiger Spiegelhalter (23, 24) realisiert ist.

4. Laserscanning-Mikroskop nach Anspruch 1, **dadurch gekennzeichnet, dass** als Zielebene (41) des mindestens einen Anregungsstrahls (11; 12) die Eintrittspupille der Fokussieroptik (44) oder eines anderen aperturbegrenzten optischen Elements gewählt ist.

5. Laserscanning-Mikroskop nach Anspruch 1, **dadurch gekennzeichnet, dass** der Strahlteiler (42) zur Erzeugung des Teststrahls (43) in Abhängigkeit von der Strahlführung des mindestens einen Anregungsstrahls (11; 12) im Messkopf (4) entweder als Reflexions- oder Transmissionsstrahlteiler ausgebildet ist.

6. Laserscanning-Mikroskop nach Anspruch 1, **dadurch gekennzeichnet, dass** der Strahlteiler (42) zur Auskopplung des Teststrahls (43) gleichzeitig als dichroitischer Spiegel zur kollinearen Einkopplung zweier unterschiedlicher Anregungsstrahlen (11; 12) ausgebildet ist.

7. Laserscanning-Mikroskop nach Anspruch 1, **dadurch gekennzeichnet, dass** die Photodiode (5) eine kreisrunde Photodiode (51) ist.

8. Laserscanning-Mikroskop nach Anspruch 1, **dadurch gekennzeichnet, dass** zusätzlich zur Regelung der Strahlstabilität in der Übertragungsoptik (3) die Pointing-Stabilität des Lasers bestimmbar und korrigierbar ist.

9. Laserscanning-Mikroskop nach Anspruch 1, **dadurch gekennzeichnet, dass** das Detektorsystem für die Sekundärstrahlung mit einem Detektor (54) zur MPT- oder SHG-Bildgebung ausgebildet ist, sodass ein flexibles Diagnosesystem für fluoreszierende Substanzen vorhanden ist, das über einen flexiblen optischen Gelenkarm (31) geringer mechanischer Genauigkeit eine beliebig freie Positionierung des Messkopfes (4) im Raum gestattet und eine reproduzierbare MPT-Bildgebung gewährleistet.

10. Laserscanning-Mikroskop nach Anspruch 1, **dadurch gekennzeichnet, dass** zwei Laserstrahlen (11, 12) unterschiedlicher Wellenlänge in einen flexiblen optischen Gelenkarm (31) eingekoppelt sind und das Detektorsystem für die Sekundärstrahlungen mit Detektoren (54; 55) zur MPT-Bildgebung und zur CARS-Bildgebung ausgestattet ist, sodass ein flexibles Diagnosesystem für fluoreszierende und nichtfluoreszierende Substanzen in lebender Materie vorhanden ist, das über einen gemeinsamen flexiblen optischen Gelenkarm (31) eine beliebig freie Positionierung des gemeinsam genutzten Messkopfes (4) im Raum gestattet und eine gleichzeitige und ortsgleiche Bildgebung aus MPT- und CARS-Signalen erzeugt.

11. Laserscanning-Mikroskop nach Anspruch 1, **dadurch gekennzeichnet, dass** zwei Laserstrahlen (11, 12) unterschiedlicher Wellenlänge in einen flexiblen optischen Gelenkarm (31) und eine separate optische Faser (32) eingekoppelt sind und das Detektorsystem mit einem gemeinsamen Detektor (57) für die Sekundärstrahlungen zur MPT-Bildgebung und zur CARS-Bildgebung ausgestattet ist, sodass ein flexibles Diagnosesystem für fluoreszierende und nichtfluoreszierende Substanzen in lebender Materie vorhanden ist, das über einen flexiblen optischen Gelenkarm (31) und eine separate optische Faser (32) eine beliebig freie Positionierung des gemeinsam genutzten Messkopfes (4) im Raum gestattet und eine gleichzeitige und ortsgleiche Bildgebung aus MPT- und CARS-Signalen erzeugt.

12. Laserscanning-Mikroskop nach Anspruch 1, **dadurch gekennzeichnet, dass** zwei Laserstrahlen (11, 12) unterschiedlicher Wellenlänge in zwei separate optische Fasern (32; 33) eingekoppelt sind und das Detektorsystem für die Sekundärstrahlungen mit Detektoren (54; 55) zur MPT-Bildgebung und zur CARS-Bildgebung ausgestattet ist, sodass ein flexibles Diagnosesystem für fluoreszierende und nichtfluoreszierende Substanzen in lebender Materie vorhanden ist, das über zwei separate optische Fasern (32; 33) eine beliebig freie Positionierung des gemeinsam genutzten Messkopfes (4) im Raum gestattet und eine gleichzeitige und ortsgleiche Bildgebung aus MPT- und CARS-Signalen erzeugt.

13. Laserscanning-Mikroskop nach Anspruch 1, **dadurch gekennzeichnet, dass** das Detektorsystem für die Sekundärstrahlung einen gemeinsamen Messdetektor (57) zur MPT-Bildgebung und zur CARS-Bildgebung aufweist, wobei Mittel zur zeitweisen Unterbrechung der CARS-Anregung vorgesehen sind, die eine Separierung der MPT-Bildgebung gegenüber einer kombinierten Signaldarstellung erlauben.

14. Laserscanning-Mikroskop nach Anspruch 1, **dadurch gekennzeichnet, dass** das Detektorsystem für die Sekundärstrahlung TCSPC-Detektoren zur MPT-Bildgebung und zusätzlich eine Triggerdiode (56) aufweist, die einen Rückseitenreflex eines für die Messsignalauskopplung vorgesehenen Strahlteilers (451) ausnutzt.

15. Laserscanning-Mikroskop nach Anspruch 1, **dadurch gekennzeichnet, dass** der Messkopf (4) mechanisch durch einen flexiblen, in beliebigen Stellungen arretierbaren Stützarm (8) an einem mobilen Grundgerät (7) abgestützt und die Übertragungsoptik (3) zwangsweise mitgeführt ist, wobei in dem Grundgerät (7) mindestens eine Strahlungsquelle (71) zur Erzeugung gepulster Anregungsstrahlung (11; 12), eine Optikeinheit (72) zur Synchronisation der gepulsten Anregungsstrahlung (11; 12) und Ausrichtung auf mindestens eine Übertragungsoptik (31; 32; 33) zum Messkopf (4) sowie die Steuereinheit (61) zur Richtungsstabilisierung der Anregungsstrahlung (11; 12) im Messkopf (4) und eine Auswerteeinheit (6) zur Verarbeitung der vom Detektorsystem (54, 55) des Messkopfes (4) übertragenen Signale für die bildgebende Darstellung mit einer Anzeigeeinheit (62) vorhanden sind.

## Claims

1. Flexible non-linear laser scanning microscope for non-invasive three-dimensional detection, containing at least one radiation source which generates at least one excitation beam of pulsed laser radiation for initiating a secondary radiation emitted by atoms and molecules, flexible transmission optics for transmitting the radiation to a measuring head having focusing optics by which the radiation is focused in a measurement object and the emitted secondary radiation is guided back and directed to at least one detector system, wherein the measuring head (4) is connected to the at least one radiation source (1; 71; 74) so as to be freely pivotable and rotatable in space and flexibly positionable so that upright microscopy, inverted microscopy and microscopy at any solid angles can be carried out,
**characterized in that**
- at least one controllable tilt mirror (2) is arranged for deflecting and aligning the at least one excitation beam (11; 12) of pulsed laser radiation of the at least one radiation source in order to align the at least one excitation beam (11; 12) in the measuring head (4) through the transmission optics (3) in such a way that the at least one excitation beam (11; 12) is arranged concentric to an aperture-limited optical element of the measuring head (44) in any position of the measuring head (4),
- a beam splitter (42) for coupling a small portion out of the at least one excitation beam (11; 12) is arranged in the measuring head (4) in front of the focusing optics (44) in order to generate a test beam (43),
- a photodiode (5) is arranged in a plane in the test beam (43) conjugate to a target plane (41), wherein a centre alignment of the test beam (43) on the photo diode (5) corresponds to the normal alignment of the excitation beam (11; 12) in the target plane, and
- position-dependent X and Y signal components (52; 53) are successively generated by deflection of the excitation beam in X and Y direction respectively, wherein the respective maximum (521; 531) of the position-dependent X and Y signal components corresponds to the centre alignment of the test beam (43) on the photodiode (5),
- a control unit (6) is provided for driving the controllable tilt mirror (2), which control unit (6), by means of the position-dependent X and Y signal components, aligns the controllable tilt mirror in such a way that the test beam is directed to the centre of the photodiode, so as to achieve a stabilization of direction of the at least one excitation beam irrespective of the magnitude of the position-dependent impairment of the transmission optics (3).

2. Laser scanning microscope according to claim 1, **characterized in that** the at least one controllable tilt mirror (2) is driven by means of at least one controllable mirror holder based on a drive principle from the group comprising capacitive, inductive or piezo actuators, stepping motors or DC motors.

3. Laser scanning microscope according to claim 1, **characterized in that** the at least one controllable tilt mirror (2) is realized by means of a two-axis mirror holder (21; 22) or by means of two single-axis mirror holders (23; 24).

4. Laser scanning microscope according to claim 1, **characterized in that** the entrance pupil of the focusing optics (44) or of another aperture-limited optical element is selected as target plane (41) of the at least one excitation beam (11; 12).

5. Laser scanning microscope according to claim 1, **characterized in that** the beam splitter (42) for generating the test beam (43) is constructed either as a reflection beam splitter or as a transmission beam splitter depending on the beam guiding of the at least one excitation beam (11; 12) in the measuring head (4).

6. Laser scanning microscope according to claim 1, **characterized in that** the beam splitter (42) for coupling out the test beam (43) is constructed simultaneously as a dichroic mirror for collinear in-coupling of two different excitation beams (11; 12).

7. Laser scanning microscope according to claim 1, **characterized in that** the photodiode (5) is a circular photodiode (51).

8. Laser scanning microscope according to claim 1, **characterized in that** in addition to the controlling of the beam stability in the transmission optics (3), the pointing stability of the laser can be determined and corrected.

9. Laser scanning microscope according to claim 1, **characterized in that** the detector system for the secondary radiation is constructed with a detector (54) for MPT imaging or SHG imaging so as to provide a flexible diagnostic system for fluorescing substances which by means of a flexible optical articulated arm (31) of low mechanical accuracy permits any free positioning of the measuring head (4) in space and ensures a reproducible MPT imaging.

10. Laser scanning microscope according to claim 1, **characterized in that** two laser beams (11, 12) of different wavelength are coupled into a flexible optical articulated arm (31) and the detector system for the secondary radiations is equipped with detectors (54; 55) for MPT imaging and for CARS imaging so as to provide a flexible diagnostic system for fluorescing and nonfluorescing substances in living matter which by means of a shared flexible optical articulated arm (31) permits any free positioning of the shared measuring head (4) in space and generates a simultaneous, co-located imaging from MPT signals and CARS signals.

11. Laser scanning microscope according to claim 1, **characterized in that** two laser beams (11, 12) of different wavelength are coupled into a flexible optical articulated arm (31) and a separate optical fiber (32), and the detector system for the secondary radiations is equipped with a shared detector (57) for MPT imaging and for CARS imaging so as to provide a flexible diagnostic system for fluorescing and nonfluorescing substances in living matter which by means of a flexible optical articulated arm (31) and a separate optical fiber (32) permits any free positioning of the shared measuring head (4) in space and generates a simultaneous, co-located imaging from MPT signals and CARS signals.

12. Laser scanning microscope according to claim 1, **characterized in that** two laser beams (11, 12) of different wavelength are coupled into two separate optical fibers (32; 33), and the detector system for the secondary radiations is equipped with detectors (54; 55) for MPT imaging and for CARS imaging so as to provide a flexible diagnostic system for fluorescing and nonfluorescing substances in living matter which by means of two separate optical fibers (32; 33) permits any free positioning of the shared measuring head (4) in space and generates a simultaneous, co-located imaging from MPT signals and CARS signals.

13. Laser scanning microscope according to claim 1, **characterized in that** the detector system for the secondary radiation has a shared measuring detector (57) for MPT imaging and for CARS imaging, wherein means are provided for periodically interrupting the CARS excitation which allow a separation of MPT imaging with respect to a combined signal display.

14. Laser scanning microscope according to claim 1, **characterized in that** the detector system for the secondary radiation has TCSPC detectors for MPT imaging and additionally a trigger diode (56) which makes use of a back reflection of a beam splitter (451) provided for coupling out measurement signals.

15. Laser scanning microscope according to claim 1, **characterized in that** the measuring head (4) is mechanically supported by means of a flexible supporting arm (8) which can be locked in any position at a mobile base device (7) and positively drives the transmission optics (3), wherein at least one radiation source (71) for generating pulsed excitation radiation (11; 12), an optics unit (72) for the synchronization of the pulsed excitation radiation (11; 12) and alignment on at least one transmission optics (31; 32; 33) to the measuring head (4) and the control unit (61) for stabilizing the direction of the excitation radiation (11; 12) in the measuring head (4), and an evaluating unit (6) for processing the signals which are transmitted from the detector system (54, 55) of the measuring head (4) for imaging display by a display unit (62) are provided in the base device (7).

## Revendications

1. Microscope à balayage laser non linéaire flexible pour la détection tridimensionnelle non invasive, comprenant au moins une source de rayonnement générant au moins un faisceau d'excitation de rayonnement laser pulsé pour déclencher un rayonnement secondaire émis par des atomes et des molécules, une optique de transmission flexible pour transmettre le rayonnement à une tête de mesure ayant une optique de focalisation, au moyen de laquelle le rayonnement est focalisé dans un objet de mesure et le rayonnement secondaire émis est renvoyé et dirigé vers au moins un système de détection, la tête de mesure (4) étant reliée à l'au moins une source de rayonnement (1; 71; 74) de manière à pouvoir pivoter et rôtir librement dans l'espace et être positionnée flexiblement de façon que la microscopie redresseur, la microscopie inverse et la microscopie sous n'importe quel angle solide peut être effectué,
**caractérisé en ce que**
- au moins un miroir basculant commandable (2) est disposé pour dévier et aligner l'au moins un faisceau d'excitation (11; 12) du rayonnement laser pulsé de l'au moins une source de rayonnement pour aligner l'au moins un faisceau d'excitation (11; 12) dans la tête de mesure (4) à travers l'optique de transmission (3) de telle sorte que l'au moins un faisceau d'excitation (11; 12) soit disposé concentriquement à un élément optique à ouverture limitée de la tête de mesure (44) dans n'importe quelle position de la tête de mesure (4),
- un séparateur de faisceau (42) pour découpler une petite portion de l'au moins un faisceau d'excitation (11; 12) est disposé dans la tête de mesure (4) devant l'optique de focalisation (44) pour générer un faisceau de test (43),
- une photodiode (5) est disposée dans un plan dans le faisceau de test (43) conjugué à un plan cible (41), un alignement central du faisceau de test (43) sur la photodiode (5) correspondant à l'alignement normal du faisceau d'excitation (11, 12) dans le plan cible, et
- des composants de signal X et Y (52; 53) dépendants de position sont successivement générés par déflection du faisceau d'excitation en direction X et Y respectivement, le maximum (521; 531) des composants de signal X et Y (52; 53) dépendants de position correspondant à l'alignement central du faisceau de test (43) sur la photodiode (5)
- une unité de contrôle (6) est prévue pour entraîner/actionner le miroir basculant commandable (2), l'unité de contrôle (6), au moyen des composants de signal X et Y (52; 53) dépendants de position, alignant le miroir basculant commandable de telle manière que le faisceau de test (43) est dirigé vers le centre de la photodiode pour réaliser une stabilisation de direction de l'au moins un faisceau d'excitation, indépendamment de la magnitude de l'impairment dépendant de position de l'optique de transmission (3).

2. Microscope à balayage laser selon la revendication 1, **caractérisé en ce que** l'au moins un miroir basculant commandable (2) est actionné/entraîné au moyen d'au moins un porte-miroir commandable sur la base d'un principe d'actionnement/entraînement à partir d'un groupe comprenant d'actionneurs capacitifs, inductifs ou piézoélectriques, de moteurs pas à pas ou de moteurs à courant continu.

3. Microscope à balayage laser selon la revendication 1, **caractérisé en ce que** l'au moins un miroir basculant commandable (2) est réalisé au moyen d'un porte-miroir biaxial (21; 22) ou de deux porte-miroirs uniaxiaux (23, 24).

4. Microscope à balayage laser selon la revendication 1, **caractérisé en ce que** la pupille d'entrée de l'optique de focalisation (44) ou d'un autre élément optique à ouverture limitée est sélectionnée en tant que plan cible (41) de l'au moins un faisceau d'excitation (11; 12).

5. Microscope à balayage laser selon la revendication 1, **caractérisé en ce que** le séparateur de faisceaux (42) pour générer le faisceau de test (43) est construit soit en tant que séparateur de faisceaux de réflexion ou en tant que séparateur de faisceau de transmission en fonction du guidage de faisceau de l'au moins un faisceau d'excitation (11; 12) dans la tête de mesure (4).

6. Microscope à balayage laser selon la revendication 1, **caractérisé en ce que** le séparateur de faisceau (42) pour découpler le faisceau de test (43) est construit en même temps en tant qu'un miroir dichroïque pour le couplage colinéaire de deux faisceaux d'excitation (11; 12) différents.

7. Microscope à balayage laser selon la revendication 1, **caractérisé en ce que** la photodiode (5) est une photodiode circulaire (51).

8. Microscope à balayage laser selon la revendication 1, **caractérisé en ce qu'**en plus du contrôle de la stabilité de faisceau dans l'optique de transmission (3), la stabilité de pointage du laser peut être déterminée et corrigée.

9. Microscope à balayage laser selon la revendication 1, **caractérisé en ce que** le système de détection pour le rayonnement secondaire est construit avec un détecteur (54) pour l'imagerie MPT ou SHG, de sorte qu'un système de diagnostic flexible pour des substances fluorescentes est présent, qui, au moyen d'un bras articulé (31) optique flexible de faible précision mécanique, permet un positionnement libre quelconque de la tête de mesure (4) dans l'espace et garantit une imagerie MPT reproductible.

10. Microscope à balayage laser selon la revendication 1, **caractérisé en ce que** deux faisceaux laser (11, 12) de longueur d'onde différente sont couplés dans un bras articulé (31) optique flexible et le système de détection pour les rayonnements secondaires est équipé de détecteurs (54; 55) pour l'imagerie MPT et pour l'imagerie CARS, de sorte qu'un système de diagnostic flexible pour des substances fluorescentes et non fluorescentes dans les matières vivantes est présent, qui, au moyen d'un bras articulé (31) optique flexible conjoint, permet un positionnement libre quelconque de la tête de mesure (4) conjointe dans l'espace et génère une imagerie simultanée et à même emplacement à partir de signaux MPT et CARS.

11. Microscope à balayage laser selon la revendication 1, **caractérisé en ce que** deux faisceaux laser (11, 12) de longueur d'onde différente sont couplés dans un bras articulé (31) optique flexible et une fibre (32) optique séparée, et le système de détection pour les rayonnements secondaires est équipé d'un détecteur conjoint (57) pour l'imagerie MPT et pour l'imagerie CARS, de sorte qu'un système de diagnostic flexible pour des substances fluorescentes et non fluorescentes dans les matières vivantes est présent, qui, au moyen d'un bras articulé (31) optique flexible et une fibre (32) optique séparée, permet un positionnement libre quelconque de la tête de mesure (4) conjointe dans l'espace et génère une imagerie simultanée et à même emplacement à partir de signaux MPT et CARS.

12. Microscope à balayage laser selon la revendication 1, **caractérisé en ce que** deux faisceaux laser (11, 12) de longueur d'onde différente sont couplés dans deux fibres (32; 33) optiques séparées et le système de détection pour les rayonnements secondaires est équipé de détecteurs (54; 55) pour l'imagerie MPT et pour l'imagerie CARS, de sorte qu'un système de diagnostic flexible pour des substances fluorescentes et non fluorescentes dans les matières vivantes est présent, qui, au moyen de deux fibres (32; 33) optiques séparées, permet un positionnement libre quelconque de la tête de mesure (4) conjointe dans l'espace et génère une imagerie simultanée et à même emplacement à partir de signaux MPT et CARS.

13. Microscope à balayage laser selon la revendication 1, **caractérisé en ce que** le système de détection pour le rayonnement secondaire a un détecteur de mesure conjoint (57) pour l'imagerie MPT et pour l'imagerie CARS, des moyens d'interruption temporaire de l'excitation CARS étant prévus, qui permettent une séparation de l'imagerie MPT par rapport à une représentation de signaux combinée.

14. Microscope à balayage laser selon la revendication 1, **caractérisé en ce que** le système de détection pour le rayonnement secondaire a des détecteurs TCSPC pour l'imagerie MPT et supplémentairement une diode de déclenchement (56) qui utilise une réflexion arrière d'un séparateur de faisceau (451) prévu pour le découplage de signaux de mesure.

15. Microscope à balayage laser selon la revendication 1, **caractérisé en ce que** la tête de mesure (4) est supportée mécaniquement par un bras de support (8) flexible qui est verrouillable dans une position quelconque sur une unité de base mobile (7) et qui entraîne positivement l'optique de transmission (3), au moins une source de rayonnement (71) pour générer un rayonnement d'excitation (11; 12) pulsé, une unité optique (72) pour synchroniser le rayonnement d'excitation (11; 12) pulsé et pour l'aligner sur au moins une optique de transmission (31; 32; 33) à la tête de mesure (4) et l'unité de contrôle (61) pour stabiliser la direction du rayonnement d'excitation (11; 12) dans la tête de mesure (4) et une unité d'évaluation (6) pour traiter les signaux transmis par le système de détection (54, 55) de la tête de mesure (4) pour la représentation d'imagerie avec une unité d'affichage (62) étant présentes dans l'unité de base (7).
